# EUROPEAN PATENT APPLICATION

(11) **EP 3 613 351 A1**
(43) Date of publication of application: **26.02.2020**
(21) Application number: 18190239.6
(22) Date of filing: 22.08.2018
(51) Int. Cl.: A61B 8/08, A61B 8/12

(54) **CORONARY CIRCULATION USING INTRA-CARDIAC ECHO**

(71) Applicant: Koninklijke Philips N.V., 5656 AE Eindhoven (NL)
(72) Inventor: HAASE, Christian, 5656 AE Eindhoven (NL); GRASS, Michael, 5656 AE Eindhoven (NL); VAN DER HORST, Arjen, 5656 AE Eindhoven (NL)
(74) Representative: de Haan, Poul Erik

(57) **Abstract**

The present invention relates to an intra cardiac echo (ICE) imaging device, an ICE imaging system and method for assessing coronary circulation using an ICE probe. An image (1) of a cardiac region is received, wherein the image provides at least one or more coronary vessels (3) and/or at least one heart chamber (5); at least one region of interest (ROI) (7) within the image is defined; imaging parameters of an ICE probe (9) are received; and at least one of a location, an orientation and access path of the ICE probe relative to the RO is calculated based on anatomical content in the ROI and/or in the cardiac region and the imaging parameters of the ICE probe, wherein the calculated location and/or orientation and/or access path is optimal for imaging of the at least one ROI.

## Description

### FIELD OF THE INVENTION

The present invention relates to an intra cardiac echo imaging device, an intra cardiac echo imaging system, a method for assessing coronary circulation using an intra cardiac echo probe and an according computer program element and a computer readable medium.

### BACKGROUND OF THE INVENTION

An intra cardiac echo (ICE) probe or catheter allows to record ultra-sound images from inside the heart chambers. For electrophysiology (EP) procedures like cardiac ablation, or resynchronization therapy aortic blood flow, a pulmonary annulus, an inner ventricular or atrial wall, or other structures are imaged from inside the heart. Furthermore, the ICE probe can be used to record Doppler ultrasound (US) images of coronary arteries of a patient's heart. Such Doppler images allow to derive coronary blood flow, which is often regarded as important measure for diagnosis and treatment of coronary artery disease.

An ideal positioning of the ICE probe for imaging of the coronary arteries is often crucial. As the coronaries might not always be visible in an image, and since the Doppler recording requires a suitable angle with respect to the blood flow, the positioning of the ICE might become a difficult task.

### SUMMARY OF THE INVENTION

There may thus be a need to provide improved positioning of the ICE probe for imaging coronary circulation.

The object of the present invention is solved by the subject-matter of the independent claims; further embodiments are incorporated in the dependent claims. It should be noted that the following described aspects of the invention apply also for the device, system, method and computer element claims.

According to the present invention, an intra cardiac echo imaging device is provided. The device comprises an image acquisition unit configured to provide an image of a cardiac region, wherein the image provides at least one or more coronary arteries and/or at least one heart chamber, and a processing unit. The processing unit is configured to define at least one region of interest within the image and calculate at least one of a location, an orientation or access path of an intra cardiac echo probe relative to the region of interest, based on imaging parameters of the intra cardiac probe and on anatomical content in the region of interest and/or in the cardiac region.

According to the present invention, also an intra cardiac echo imaging system is provided. The system comprises an intra cardiac imaging (ICE) probe and an intra cardiac echo imaging device as described above. The imaging of the coronary arteries with an ICE probe aids to reduce the need for X-ray radiation and reduce the amount of administered contrast agents.

According to an example, the system further comprises a trans-esophageal echo or a trans-thoracic echo. Navigation of the ICE probe can be optionally guided by an additional trans-esophageal echo or a trans-thoracic echo (TEE, TTE).

According to an example, the system further comprises a robotic steering device. The navigation of the ICE probe may also be supported by robotic steering replacing or supporting the operator or user.

According to the present invention, also a method for assessing coronary circulation using an intra cardiac echo (ICE) probe is provided. The method comprises the following steps: acquiring an image of a cardiac region, wherein the image provides at least one or more coronary arteries and/or at least one heart chamber; defining at least one region of interest within the image; calculating at least one of a location, orientation or access path of the ICE probe relative to the region of interest based on imaging parameters of the ICE probe and anatomical content in the region of interest and/or in the cardiac region.

The ICE probe comprises an ultrasound transducer. This may be a phased array and Doppler capable transducer for the assessment of epicardial and intramyocardial coronary blood flow.

The provided method for assessing coronary circulation allows imaging of the coronary circulation using the ICE probe. The method encompasses guidance measures to allow optimal intra-cardiac placement of the ICE probe for the imaging of the at least one region of interest within the image, comprising the coronary circulation. Furthermore, the method also encompasses processing, allowing to obtain, for example, a flow velocity or a perfusion score from the measurement data acquired by the ICE probe. Furthermore, a fluid dynamic model is created based on image data of a cardiac region and diagnostic data.

The provided imaging of the coronary arteries with an ICE probe aids to reduce the need for X-ray radiation and to reduce the amount of administered contrast agents.

Acquiring an image of a cardiac region comprises recording of such an image, e.g. by cardiac CT angiography, MR angiography, ultrasound imaging, wherein the image is provided as a two- or three-dimensional (2D or 3D) image. The image can also be provided from a data base, for example, or generated from provided image data. The image comprises a diagnostic image, providing at least one or more coronary vessels and/or at least one heart chamber. The image is provided as image data to an image acquisition unit. The image of a cardiac region may also be a general, virtual, or abstract anatomical model of the coronary anatomy.

At least one region of interest (ROI) is defined within the image. The one or more regions of interest are defined as regions in the coronary circulation of the heart that are to be investigated or examined. In an example a specific coronary branch with a narrowing is investigated or examined. In another example an area of the coronary microcirculation that is suspected to be ischemic is investigated or examined.

At least one of a location, orientation of the intra cardiac echo probe relative to the region of interest or the access path of the ICE probe is calculated. In some examples more than one location, orientation and/or access path of the ICE probe, or combinations therefrom, are comprised by the calculating step of the method. The calculated location and/or orientation and/or access path is optimal for imaging of the at least one region of interest.

An optimal ICE probe positioning, providing the location, orientation and access path, is based on anatomical content in the cardiac image and/or the ROI within the image and the imaging capabilities of the ICE probe. The anatomical content comprises coronary vessels, heart chambers (ventricles and atria), myocardial tissue, implants, external devices, and or any connected parts of the patient's anatomy.

Such optimal locations and/or orientations of the probe could be bound to locations from which the ROI is actually visible in the US field of view and which can also be accessed by the ICE probe, e.g. inside a ventricle.

Calculating the location and/or orientation and/or an access path of the intra cardiac echo probe relative to the ROI is derived from the image of the cardiac region or from the ROI within the image. In some examples, image data provided by the ICE probe is used for tracking of the probe. In various examples, the method includes displaying the calculated location, orientation or access path, e.g. on a provided display.

According to an example, the imaging parameters comprise at least one of a field of view, an acquisition mode, an angle of orientation or an accessibility of an access path of the intra cardiac echo probe. Exemplary criteria for defining the imaging parameters could be the accessibility of the location by a provided ICE catheter from a navigation point of view. Also, the angle of orientation of the ICE probe is influenced by the blood flow direction in the coronary artery, so that a sufficiently flat angle relative to the probe is required to allow Doppler imaging. Thus, the angle of orientation of the probe can be defined as with respect to the blood flow. The accessibility of the access path may follow from the location and/or orientation. For examples, if the location is determined in the left ventricle, a different path may be suggested than for a location determined in the right ventricle.

In various examples, the region of interest would be a segment of a coronary artery and the positioning of the ICE probe might be limited to the left ventricle (above referred to as anatomical content) and the field of view of the ICE probe (above referred to as imaging parameter of the ICE probe), which is naturally limited by the ultrasound cone of the probe. In some examples, optimal position(s) and orientation(s) of the probe would be limited to locations/orientations from which the at least one region of interest (ROI) is actually visible in the US field of view and which are also located in the ventricle. In an example, heart phase dependent deformations may be considered to allow optimal blood flow imaging during a specific heart phase, e.g. the diastole.

In some examples, the left side of the heart will often be accessed via the aorta. The right side of the heart via the venous system. On the other hand, the left atrium is often easier to access via trans-septal puncture, i.e. moving via the venous system in the right atrium and puncturing the septal wall to gain access. The puncture location of the septal wall may depend on patient anatomy, or comorbidities. Thus, the regions that can be easily reached in the heart chambers, and the easiest way to reach them may vary from patient to patient.

According to an example, at least one of the calculated location, orientation or access path of the probe is displayed comprising a road map. The road map along with the location, orientation and/or access path of the ICE probe is displayed to an operator in a catheter lab, for example. In an example, the road map comprises a road map overlay on 2D fluoroscopy images. In another example, the road map comprises a 3D road map in combination with 3D ICE catheter tracking.

According to an example, at least one of the calculated location, orientation or access path of the probe is provided by color-coding. Feedback to the user can be given by the color-coding indicating if the probe is at the correct or desired location. In an example, the probe is colored green when it reaches the calculated location and orientation; the probe is colored orange when reaching the calculated location and the orientation is substantially different from the calculated orientation.

According to an example, the calculated location and/or orientation of the intra cardiac echo probe is provided along with tracking of a three-dimensional location of the probe. The location and/or orientation of the ICE probe in an optimal location for imaging of the at least one region of interest is provided. The location and/or orientation is tracked providing a 3D location of the ICE probe with respect to the patient's anatomy, for example. In some examples, tracking of the location and/or orientation of the intra cardiac echo probe is provided in relation to identified structures within the image of a cardiac region. Tracking of the probe relative to the previously acquired 3D image may also be performed based on registering the live US data to the 3D image. Other tracking techniques of the ICE probe, such as electromagnetic or magnetic tracking along with co-registration on the image of the cardiac region, well known in the art, may also be used.

According to an example, diagnostic parameters are defined based on image data of the image of a cardiac region, and the tracking of a three-dimensional location and/or orientation of the probe. Diagnostic parameters may also be defined from the ICE data by using the (previously acquired) image of a cardiac region, e.g. 3D anatomy data, and the tracked 3D location and/or orientation of the ICE probe. In an exemplary example, the ICE probe was moved to the identified (calculated) location, e.g. is now green in the overlay, optionally an operator could confirm correct positioning, and then the diagnostic parameters are calculated based on the assumption that the probe is correctly positioned. In another exemplary example, the diagnostic parameters are calculated based on the tracked 3D location and/or orientation of the ICE probe, together with the relative information of the 3D anatomy data.

The diagnostic parameters comprise coronary blood flow velocity which may be determined from US Doppler data taking into account the angle (theta) between the blood flow and an ultrasound transducer on the ICE probe. In an example, coronary flow reserve (CFR) (ratio of flow in hyperemia and rest) can be determined using the US Doppler data in rest and hyperemia and taking into account the difference in the angle theta between the measurements in rest and hyperemia. Also, the measured diagnostic parameters may be automatically registered to the corresponding regions in the image of a cardiac region. The diagnostic parameters comprise implementation of diagnostic image processing (e.g. perfusion analysis) in the US image, wherein the provided 3D probe location and/or orientation and the information provided by the image of a cardiac region, e.g. 3D anatomy information, is used to substantially bound the processing to the defined at least one region of interest.

According to an aspect of the invention, diagnostic parameters can be provided using an ICE probe by providing a method comprising the following steps: acquiring an image of a cardiac region, wherein the image provides at least one or more coronary arteries and/or at least one heart chamber; defining at least one region of interest within the image; providing diagnostic parameters based on tracking of a three-dimensional location and/or orientation of the ICE probe and anatomical content in the region of interest and/or in the cardiac region.

In some examples, the ICE probe may have a wide field of view and is placed at a location (somewhere) in the heart. Using beam steering of the US beam of the ICE probe, imaging is (automatically) focused on the ROI within the image of a cardiac region. The US acquisition mode or image processing is based on the anatomical data in the image of a cardiac region. For example, if there is a lot of dense tissue in-between the probe and the ROI, a different US frequency may be used. Also, anatomy information can be used to "clean up" the displayed US image. CFR may be automatically calculated in the ROI.

Image acquisition, image processing or image analysis may be adapted and/or defined based on image data of the image of a cardiac region and the tracking of the three-dimensional location and/or orientation of the ICE probe.

According to the present invention, also a method for creating a fluid dynamic model for coronary circulation using an intra cardiac echo probe, the method comprising the following steps: acquiring an image of a cardiac region, wherein the image provides at least one or more coronary arteries, providing diagnostic data comprising image data provided by the intra cardiac probe, and creating the fluid dynamic model. Image data of the image of a cardiac region is used in combination with the diagnostic data to create the fluid dynamics model and/or for calculating diagnostic parameters using the fluid dynamic model.

Acquiring an image of a cardiac region comprises recording of such an image, e.g. by cardiac CT angiography, wherein the image is provided as a two- or three-dimensional (2D or 3D) image. The image can also be provided from a data base, for example, or generated from provided image data. The image comprises a diagnostic image, providing at least one or more coronary arteries.

The diagnostic data comprises image data provided by the ICE probe. For example, the geometry of the coronary arteries in an angiogram (the image of a cardiac region) is segmented. From this information of artery geometry a model of vessel resistances can be derived that can be used for fluid dynamic modelling. The boundary conditions (e.g. the flow rate in this model of vessel resistances) may be provided or adapted based on the blood flow data provided by the ICE probe. Diagnostic image data also comprises refining or adapting a geometric model of the coronary arteries based on the ICE data while leaving the flow boundary conditions unchanged. Diagnostic data also comprises aortic pressure data received from a contrast catheter or intravascular pressure data received from a pressure wire or diagnostic data related to coronary circulation.

Furthermore, data derived from the ICE probe imaging can be used as input boundary condition in the fluid dynamics model for the calculation of diagnostic parameters like instantaneous wave free ratio (iFR), fractional flow reserve (FFR), hyperemic stenotic resistance (HSR), index of microvascular resistance (IMR), hyperemic microvascular resistance (HMR). For example, a measured blood flow velocity or a perfusion analysis may provide information about resistance in the vessel or of the microvasculature.

A fluid dynamic model may indicate critical region in the coronary circulation, e.g. a lesion with an expected FFR<0.8. This may result in the recommendation to perform ICE based flow analysis in the vessel with an expected FFR<0.8.

According to an example, the fluid dynamic model is used for defining the at least one region of interest. Using the fluid dynamic model for defining the region of interest for intra cardiac echo imaging further improves assessing coronary circulation.

Optionally, for an ultrasound (US) based perfusion analysis of the microvasculature but also for coronary flow velocity imaging an US contrast agent may be used. The US contrast agent would provide better visibility of the coronary arteries in the US image data. This would lead to more accurate measurement of diagnostic parameters (E.g. by Doppler flow). A quantitative perfusion analysis of a tissue region is possible with contrast agent. First, an image before injection is compared with at least one image after injection of the contrast agent. The increase in contrast can be measured by subtracting the two images. The increase in contrast is related to the amount of contrast agent perfusing the tissue in the time between the two image acquisitions. This is related to the general tissue perfusion with blood. With multiple images after injection the time intensity curve may be used to measure how quickly the tissue is perfused by the contrast agent. This comprises the time for reaching the peak contrast, and/or the time it takes for the contrast agent to be washed out of the tissue. This is all related to measure or quantify tissue perfusion with blood.

According to an aspect, a computer program element is provided for controlling an apparatus as described above, which, when being executed by a processing unit, is adapted to perform the method steps described above. A computer readable medium having stored the program element thereon is provided.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1: an image of a cardiac region;
Fig. 2: a flowchart of the method providing guidance measures;
Fig. 3: a flowchart of the method providing processing measures;
Fig. 4: a flowchart of the method providing a fluid dynamic model;
Fig. 5: an ICE imaging device;
Fig. 6: an ICE imaging system.

### DETAILED DESCRIPTION OF THE EMBODIMENTS

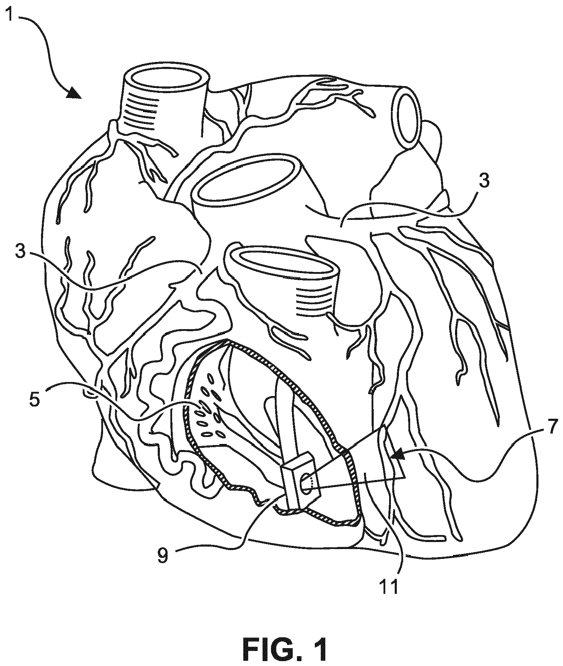

Fig. 1 shows an image 1 of a cardiac region providing coronary arteries 3. A heart chamber 5 is visible in the image 1. The region of interest (ROI) 7 is provided within the image 1. The ICE probe 9 is located within the heart chamber 5 providing an US imaging cone 11.

The above described features, functionalities and properties with regard to method, device and system also apply for the features shown and described with respect to Fig. 1 and are not repeated again.

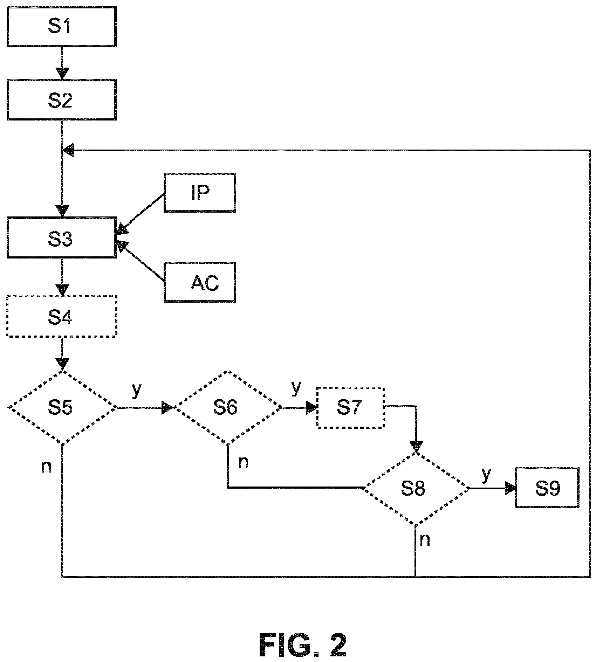

Fig. 2 shows a flowchart of the method providing guidance measures allowing optimal intra-cardiac placement of the ICE probe for the imaging of an ROI within an image comprising coronary circulation, for example.

An image of a cardiac region is acquired S 1. The image provides at least one or more coronary vessels and/or at least one heart chamber. At least one ROI is defined S2 within the image. At least one of a location, orientation or access path of the ICE probe relative to the ROI is calculated S3 based on imaging parameters IP of the ICE probe and anatomical content AC in the ROI and/or in the cardiac region.

In some embodiments, the calculated location, orientation and/or access path of the ICE probe may be displayed S4 on a display or otherwise provided to an operator. If the calculated location, orientation and/or access path of the ICE probe is accepted S5, e.g. by an operator or otherwise, a roadmap could be required S6 and displayed as overlay S7 on the ROI. If no roadmap is required S6, color-coding could be required S8. If color-coding is required S8 at least the ROI providing color-coding is displayed S9. In some embodiments, a roadmap and color-coding is displayed S9.

In case the calculated location, orientation and/or access path of the ICE probe is not accepted S5 a new location, orientation and/or access path of the ICE probe is calculated.

The above described features, functionalities and properties with regard to method, device and system also apply for the features shown and described with respect to Fig. 2 and are not repeated again.

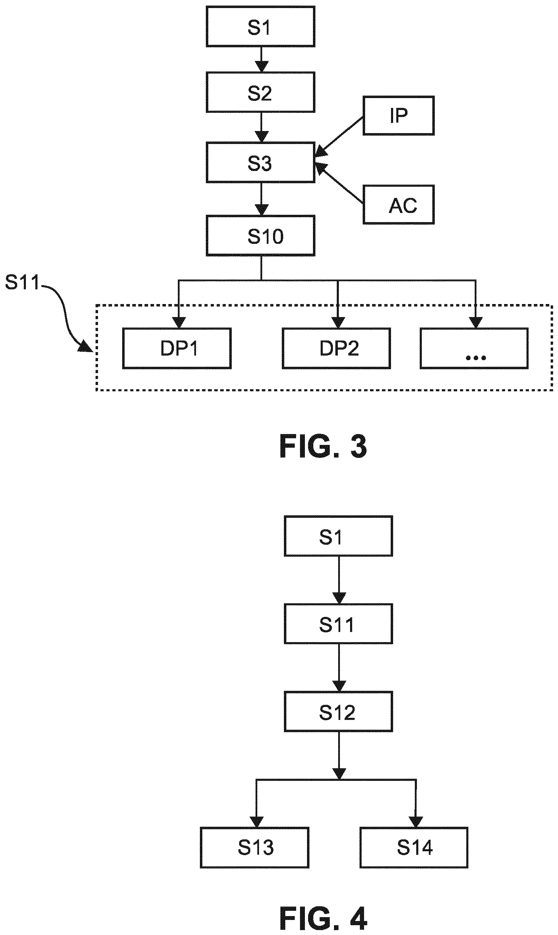

Fig. 3: a flowchart of the method providing processing measures, allowing to obtain, for example, a flow velocity or a perfusion score from the measurement data acquired by the ICE probe.

As in Fig. 1 an image of a cardiac region is acquired S1. The image provides at least one or more coronary arteries and/or at least one heart chamber. At least one ROI is defined S2 within the image. At least one of a location, orientation or access path of the ICE probe relative to the ROI is calculated S3 based on imaging parameters IP of the ICE probe and anatomical content AC in the ROI and/or in the cardiac region.

The ICE probe is placed S10 in an optimal position for investigating the ROI. The optimal position is defined by the calculated location and/or orientation and/or access path of the ICE probe, as described above.

One or more diagnostic parameters DP(1..n) are calculated S11 from the data acquired by the ICE probe based on image data of the image of a cardiac region and tracking of a three-dimensional location and/or orientation of the probe. The diagnostic parameters may include coronary blood flow velocity, or coronary flow reserve using US Doppler data.

The above described features, functionalities and properties with regard to method, device and system also apply for the features shown and described with respect to Fig. 3 and are not repeated again.

Fig. 4 shows a simplified schematic of a flowchart of a method providing a fluid dynamic model for coronary circulation using an ICE probe.

An image of a cardiac region is acquired S1, wherein the image provides at least one or more coronary vessels. The image may be a CT angiography, MR angiography, ultrasound image. Diagnostic data of the cardiac region is extracted from the image data, which may include at least one of a coronary blood flow velocity, coronary flow reserve. A fluid dynamic model is created based on the image data of the cardiac region (e.g. morphology of the vessel such as vessel diameter, length) in combination with diagnostic data (e.g. a coronary blood flow velocity, coronary flow reserve, FFR) of the cardiac region extracted from the image data. In an example the fluid dynamic model may indicate critical region in the coronary circulation, e.g. a lesion with an expected FFR<0.8. The FFR may be ascertained from flow velocities along the vessel determined directly from the angiography image of the cardiac region, or from pressure calculations along the vessel by using the fluid dynamic model. This may result in the recommendation to perform ICE imaging with the ICE probe based on the flow analysis in the vessel with an expected FFR<0.8. Therefore, the fluid dynamic model can be used for defining S13 at least one ROI. In an alternative or optional embodiment, diagnostic parameters can be determined S14 by using the fluid dynamic model, such as blood pressure in the vessel or resistance of the vessel. When the ICE probe is placed in an optimal position for investigating the ROI according to the calculated location and/or orientation and/or access path of the ICE probe, one or more diagnostic parameters DP(1..n) can be derived from the ultrasound data acquired by the ICE probe. The diagnostic parameters may include coronary blood flow velocity, or coronary flow reserve using US Doppler data. The diagnostic parameters DP(1..n) may further be used to create a fluid dynamic model based on image data of the image of a cardiac region or the ROI, in order to further determine other diagnostic parameters such as vessel resistance, blood pressure, FFR, which provide a refined and more accurate measurement/ascertainment of diagnostic parameters with respect those that can be derived from the image of the cardiac region acquired in S1 or the fluid dynamics model created based on the angiography image.

The above described features, functionalities and properties with regard to method, device and system also apply for the features shown and described with respect to Fig. 4 and are not repeated again.

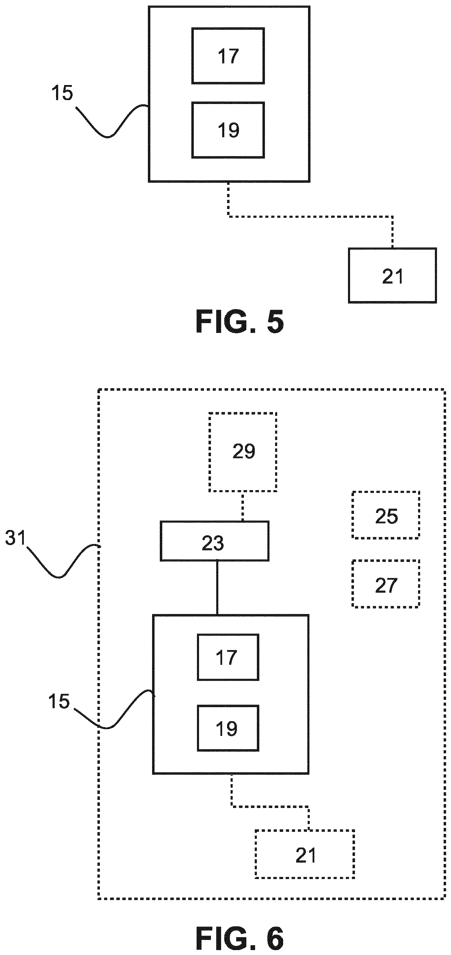

Fig. 5 shows an ICE imaging device 15. The ICE imaging device 15 comprises an image acquisition unit 17 and a processing unit 19. The image acquisition unit 17 is configured to receive or provide an image of a cardiac region, wherein the image provides at least one or more coronary vessels and/or at least one heart chamber. The processing unit 19 is configured to define at least one region of interest within the image and to calculate at least one of a location, an orientation or access path of the intra cardiac echo probe relative to the region of interest based on imaging parameters of the intra cardiac echo probe and anatomical content in the region of interest and/or in the cardiac region.

In some embodiments the ICE imaging device 15 is connectable to a display or display unit 21 to display measured or calculated entities and/or parameters as described with reference to Figs. 1 to 4.

The above described features, functionalities and properties with regard to method, device and system also apply for the features shown and described with respect to Fig. 5 and are not repeated again.

Fig. 6 shows an ICE imaging system 31 providing an ICE probe 23 and an intra cardiac echo imaging device 15 as in Fig. 5. In some embodiments the system may comprise a trans-esophageal echo 25 probe or a trans-thoracic echo 27 probe. In some embodiments, the system also provides a robot steering device 29 for navigating the ICE probe toward the location and/or orientation that is calculated according to S3 and accepted by the operator according to S5. Feedback to the user can be given by the color-coding indicating if the probe is at the correct or desired location and/or orientation and/or whether the access path of the ICE probe is correctly followed. In an example, the probe is colored green when it reaches the calculated location and orientation; the probe is colored orange when the location and/or orientation of the ICE probe is substantially different from the calculated location and/or orientation. In an alternative embodiment the ICE probe can be represented differently when it follows correctly the access path that is calculated for the desired location to optimally image the ROI, when it actually reaches the desired location and/or orientation and when its actual location and/or orientation is substantially deviating from the desired access path and/or desired location. The different representation may be according to different colors. In a further embodiment a color can be used when the ICE probe reaches the desired location and is still not in the desired orientation, and a different color when both the actual location and orientation match the desired ones.

The above described features, functionalities and properties with regard to method, device and system also apply for the features shown and described with respect to Fig. 6 and are not repeated again.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An intra cardiac echo imaging device (15), comprising:
an image acquisition unit (17) configured to receive an image (1) of a cardiac region, wherein the image provides at least one or more coronary vessels and/or at least one heart chamber;
a processing unit (19) configured to:
define at least one region of interest (7) within the image (1);
receive imaging parameters of an intra cardiac echo probe (9,23);
calculate at least one of a location, an orientation and access path of the intra cardiac echo probe relative to the region of interest based on:
anatomical content in the region of interest and/or in the cardiac region;
the imaging parameters of the intra cardiac echo probe.

2. An intra cardiac echo imaging system (31), comprising:
the intra cardiac imaging probe (9,23); and
the intra cardiac echo imaging device (15) according to claim 1.

3. System according to claim 2, wherein the system further comprises a trans-esophageal echo probe (25) or a trans-thoracic echo probe (27).

4. System according to claim 2 or 3, wherein the system further comprises a robotic steering device (29).

5. System according to any of the claims 2 to 4, wherein the imaging parameters comprise at least one of a field of view, an acquisition mode, an angle of orientation and an accessibility of an access path of the intra cardiac echo probe.

6. System according to any of the claims 2 to 5, further comprising a display (21), and wherein the at least one of the calculated location, orientation and access path of the intra cardiac echo probe is displayed comprising a road map.

7. System according to claim 6, wherein the at least one of the calculated location, orientation and access path of the intra cardiac echo probe is displayed comprising a road map.

8. System according to claim 6 or 7, wherein the at least one of the calculated location and/or orientation of the intra cardiac echo probe is provided along with a three-dimensional location and/or orientation tracking of the intra cardiac echo probe.

9. System according to claim 8, wherein a representation of the intra cardiac echo probe on the display is provided differently:
when at least one of the tracked location, orientation and access path of the intra cardiac echo probe matches the respective at least one of the calculated location, orientation and access path; and
when at least one of the tracked location, orientation and access path of the intra cardiac echo probe substantially differs from the respective at least one of the calculated location, orientation and access path.

10. System according to claim 9, wherein the different representation of the intra cardiac echo probe comprises color-coding.

11. System according to any of the preceding claims, wherein the processor is configured to define the at least one region of interest (7) within the image (1) based on a fluid dynamic model created based on the image data of the cardiac region in combination with diagnostic data of the cardiac region extracted from the image data.

12. System according to any of the preceding claims, wherein the intra cardiac echo probe is configured to provide ultrasound image data of the region of interest.

13. System according to claim 12, wherein the processor is configured to derive one or more diagnostic parameters (DP) from the ultrasound image data provided by the intra cardiac echo probe.

14. Method of assessing coronary circulation by an intra cardiac echo probe, comprising:
receiving an image (1) of a cardiac region, wherein the image provides at least one or more coronary vessels and/or at least one heart chamber;
defining at least one region of interest (7) within the image (1);
receiving imaging parameters of an intra cardiac echo probe (9,23);
calculating at least one of a location, an orientation and access path of the intra cardiac echo probe relative to the region of interest based on:
anatomical content in the region of interest and/or in the cardiac region;
the imaging parameters of the intra cardiac echo probe.

15. A computer program element for controlling an intra cardiac echo imaging device (15) according to any of the claims 1 to 13, which, when being executed by a processing unit, is adapted to perform the method of claim 14.
